# EUROPEAN PATENT APPLICATION

(11) **EP 4 201 360 A1**
(43) Date of publication of application: **28.06.2023**
(21) Application number: 21306946.1
(22) Date of filing: 27.12.2021
(51) Int. Cl.: A61B 34/20, A61B 34/30, A61B 17/00

(54) **ELECTROMAGNETIC LOCALIZATION SYSTEM**

(71) Applicant: MinMaxMedical, 38400 Saint-Martin-d'Hères (FR)
(72) Inventor: LAVALLEE, Stéphane, 38400 SAINT MARTIN D'HERES (FR); ROUSSEAU, Sandra, 38400 SAINT MARTIN D'HERES (FR); SEDNAOUI, Thomas, 38400 SAINT MARTIN D'HERES (FR); HUGUEL, Loïc, 38400 SAINT MARTIN D'HERES (FR)
(74) Representative: Regimbeau

(57) **Abstract**

The present invention concerns an electromagnetic localization system (1) comprising at least one locating unit (10) and one processing unit (5), the locating unit (10) comprising at least two locating transducers (A, AA) wherein at least one locating transducer is a transmitter adapted to emit at least one magnetic field and at least one locating transducer is a receiver adapted to receive and measure the magnetic field emitted by the transmitter, wherein at least one first locating transducer (A) is adapted to be locked in a fixed position with respect to a first object (100) and at least one second locating transducer (AA) is adapted to be locked in a fixed position with respect to a second object (110), the processing unit (5) being configured to:
a. determine a pose of the first locating transducer (A) with respect to the second locating transducer (AA) based on the measurement of the magnetic field emitted by the transmitter of the locating unit,
b. calculate an indicator related to the accuracy of the measurement of the magnetic field,
c. compare the calculated indicator with a predefined threshold,
d. determine the pose of the first object (100) with respect to the second object (110), based on the determined pose of the transducers when the calculated indicator is below the predefined threshold, and
e. generate an instruction to displace at least one of the locating transducers (A, AA) when the calculated indicator exceeds the predefined threshold, such instruction being executable manually by a user of the system or automatically by an actuator.

## Description

### FIELD OF THE INVENTION

The present invention relates to the field of electromagnetic localization systems adapted to determine the pose of objects with respect to one another, and the invention especially relates to a system adapted to evaluate the reliability of the determined pose.

### BACKGROUND OF THE INVENTION

Computer-assisted surgery, surgical navigation, and surgical robotics all rely on the measurement of the relative position and orientation between several trackers attached to anatomical structures or bones, to surgical instruments, to robot ends, or to various sensors.

Optical localization systems are well known and widely used, though limited by the bulkiness of their large trackers and line of sight major constraints. Electromagnetic systems are particularly adapted to be used during minimally invasive surgeries, as they allow an electromagnetic transducer to be inserted into the patient through an incision, and as they are not limited by any line-of-sight issue.

In the present application, the word "transducer" refers to a device adapted to either emit at least one electromagnetic field or receive such an electromagnetic field.

An electromagnetic localization device generally consists of a transmitting device, also called transmitter or field generator, consisting of one or more transmitting coils rigidly linked to each other, and of a receiving device, called receiver, consisting of one or more receiving coils rigidly linked to each other. As well known in the art, the joint analysis and modeling of the magnetic fields emitted by the transmitting coils and the fields measured by the receiving coils makes it possible to determine the position and / or the orientation of the receiving device with respect to the transmitting device. Consequently, the respective position and/or orientation of objects linked to each of the receiving device and of the transmitting device can be determined as long as the geometry of the links between said transmitting or receiving devices and the objects to be tracked are known.

Typically, a transmitter comprises three coils and a receiver comprises three coils. For instance, the transmitter can comprise larger coils than the receiver. Therefore, nine measurements are obtained, and the six independent degrees of freedom of the position and orientation of a transform matrix between the receiver reference system and the transmitter reference system can be determined. In many systems, it is possible to use one transmitter and several receivers, in order to determine the relative positions and orientations of these receivers with respect to each other. For example, one receiver can be attached to a bone, and another one to the tip of an instrument, so that the relative pose of the bone with respect to the tip of the instrument can be determined.

Electromagnetic localization devices are well known in the literature, as for example in the document "Magnetic Position and Orientation Tracking System" by Frederick H. Raab et al. published in IEEE Transactions on Aerospace and Electronic Systems VOL. AES-15, No. 5 September 1979, or the document " La localisation spatiale d'outils chirurgicaux par systèmes électromagnétiques alternatifs. Applications et domaines de validite des modélisations numériques" by Joffrey Paille - Thesis, Universite Joseph-Fourier - Grenoble I, 2004. HAL Id: tel-00006125, version 1.

However, magnetic localization devices, also referred to as electromagnetic localization devices, may be prone to disturbances of the magnetic field between the transmitter and the receiver:
- Electrically conductive materials can give rise to eddy currents when these materials are placed in a varying magnetic field. The eddy currents that then circulate in this disturbing material in turn generate a disruptive magnetic field,
- Magnetic materials (for example: ferromagnetic, diamagnetic, paramagnetic, antiferromagnetic, ferrimagnetic) can distort magnetic field they are placed in. This distorted field leads to incorrect measurement.

In a typical operating room, there are many disturbing materials. Most of the surgical instruments for instance are made of such disturbing materials. In typical applications of surgical robotics and surgical navigation, the average distance between the surgical instrument and the anatomical structure to be treated can be up to 30 cm or even 50 cm. Furthermore, to achieve the level of precision expected for a surgical procedure, the distance between each of the transmitter and the receiver and the closest disturbing material should be at least 4 or even 5 times the working distance of the electromagnetic localization device which corresponds to a distance measured between the transmitter and the receiver of a same electromagnetic localization device. This means, for example, that the floor and ceiling of a room of standard height can be problematic for measuring a working distance of 30 cm or more as the distance between the operating table and the floor or the ceiling is, in a typical operating room, inferior to about 1 or 2 meter(s). Even if the working distance of the electromagnetic localization device is reduced to 15 cm, the closest disturbing material must be at a distance of at least 75 cm from the transmitter and from the receiver of such electromagnetic localization device, which is difficult to warranty.

Currently used electromagnetic localization devices are thus not adapted to be used in surgical robotics and surgical navigation. There have been many attempts to overcome these issues.

Document US7957925 describes a method of compensation of measurement artifacts generated by disturbing materials.

Document US20170202627 describes an electromagnetic system for tracking a surgical tool comprising a plurality of subsets of field generator coils disposed along edge portions of a surgical bed.

Document WO2020/120901 describes a method which ensures that the locating transducers of an electromagnetic localization system are in a measurement zone, such a measurement zone being defined as a zone wherein the introduction of a metallic object does not disturb the measurements. Thus, the authorized movements of the user of such method are limited as he/she must work in said defined zone.

However, the solutions of the prior art are not satisfactory, since they offer only partial solutions and many applications still cannot use the existing electromagnetic localization systems which lack reliability due to their sensitivity to the presence of disturbing materials.

The present invention aims at resolving at least these drawbacks by proposing an electromagnetic localization system adapted to determine whether the magnetic field is disturbed or not and to emit instructions aiming at lessening the impact of disturbing materials on the measurement of the magnetic field when a disturbance is detected.

### SUMMARY OF THE INVENTION

An object of the invention thus concerns an electromagnetic localization system comprising at least one locating unit and one processing unit, the locating unit comprising at least two locating transducers wherein at least one locating transducer is a transmitter adapted to emit at least one magnetic field and at least one locating transducer is a receiver adapted to receive and measure the magnetic field emitted by the transmitter, wherein at least one first locating transducer is adapted to be locked in a fixed position with respect to a first object and at least one second locating transducer is adapted to be locked in a fixed position with respect to a second object, the processing unit being configured to:
a. determine a pose of the first locating transducer with respect to the second locating transducer based on the measurement of the magnetic field emitted by the transmitter of the locating unit,
b. calculate an indicator related to the accuracy of the measurement of the magnetic field,
c. compare the calculated indicator with a predefined threshold,
d. determine the pose of the first object with respect to the second object, based on the determined pose of the transducers when the calculated indicator is below the predefined threshold, and
e. generate an instruction to displace at least one of the locating transducers when the calculated indicator exceeds the predefined threshold, such instruction being executable manually by a user of the system or automatically by an actuator.
In the present document, the words "locked in a fixed position" mean that the corresponding objects are in a fixed position, at least temporarily, as at least one of the locating transducers must remain mobile.

According to an embodiment of the invention, the electromagnetic localization system comprises at least one alert device and the processing unit is adapted to send the instruction to displace one of the locating transducers to the alert device, the execution of such instruction resulting in that the alert device provides an information to the user according to which one of the locating transducers must be displaced. According to this embodiment, the displacement of the locating transducer is realized manually by the user of the system who knows he/she must displace such transducer to improve the measurement thanks to said alert device.

According to an aspect of the invention, the second locating transducer is movably attached to the second object. For instance, the second locating transducer is mounted on a guide fixedly attached to the second object, and the system comprises at least one locking device adapted to lock the pose of the second locating transducer with respect to the second object. Alternately, the second locating transducer is mounted on a guide fixedly attached to the second object, the guide comprising an indexing device adapted to lock the pose of the second locating transducer in one of a number of determined poses with respect to the second object. Again, the words "lock the pose" here mean that such pose is at least temporarily locked. Optionally, the second locating transducer is motor-driven, the processing unit being adapted to send the instruction to displace one of the locating transducers to the motor driving the second locating transducer, the execution of the instruction resulting in a displacement of the second locating transducer.

According to an embodiment of the invention, the electromagnetic localization system comprises at least two locating units, each locating unit comprising at least two locating transducers, wherein at least one locating transducer is a transmitter adapted to emit at least one magnetic field and at least one locating transducer is a receiver adapted to receive and measure the magnetic field emitted by the transmitter, wherein the two locating units comprise at least one shared locating transducer adapted to be locked in a fixed position with respect to both objects and wherein the processing unit is adapted to
a. determine a pose of the locating transducers of each locating unit with respect to one another based on the measurement of the magnetic field emitted by the transmitter of the concerned locating unit,
b. calculate one indicator related to the accuracy of the measurements of the magnetic field for each measurement,
c. compare each of the calculated indicators with a predefined threshold,
d. determine the pose of the first object with respect to the second object, based on the determined pose of the transducers when the calculated indicators are both below the predefined threshold, and
e. generate an instruction to displace at least one of the locating transducers when at least one of the calculated indicators exceeds the predefined threshold, such instruction being executable manually by a user of the system or automatically by an actuator.

According to this embodiment of the invention, the processing unit is adapted to generate an instruction to displace the shared locating transducer when both calculated indicators exceed the predefined threshold. The shared locating transducer is thus temporarily locked in the fixed position with respect to both objects so as to permit its displacement when the indicator exceeds the predefined threshold.

According to another aspect of the invention, at least one of the locating units comprises at least two second locating transducers adapted to be locked in position with respect to the second object, the two second locating transducers being either transmitters adapted to emit the electromagnetic field then received and measured by the first locating transducer or receivers adapted to receive and measure the electromagnetic field emitted by the first locating transducer, the processing unit being adapted to:
a. determine the pose of the first locating transducer with respect to each of the second locating transducers,
b. calculate at least one indicator related to the accuracy of the measurements of the magnetic fields for each determined pose,
c. compare each calculated indicator to the predefined threshold
d. determine the pose of the first object with respect to the second object when at least one of the calculated indicators is below the predefined threshold, based on the determined pose of the first locating transducer with respect to the one of the second locating transducers for which the calculated indicator related to the accuracy of the measurement of the magnetic field is the lowest
e. generate an instruction to displace at least one of the locating transducers when all the calculated indicators exceed the predefined threshold, such instruction being executable manually or automatically.

According to an aspect of the invention, the processing unit can be further configured to:
a. determine a distance between the first locating transducer and the second locating transducer of at least one locating unit,
b. compare the determined distance measured between the first locating transducer and the second locating transducer of the locating unit with a predetermined threshold,
c. generate an instruction to displace at least one of the locating transducers when the determined distance exceeds the predetermined threshold, such instruction being executable manually by a user of the system or automatically by an actuator.

Such threshold can for instance be set at 60 mm. Advantageously, the threshold is set at 50 mm.

The present invention also concerns a computer-assisted surgery system, comprising at least one robotic arm with at least one motor-driven joint formed between two adjacent segments, the robotic arm extending between a base and an end-effector, such end-effector being adapted to hold a surgical guide and/or a surgical instrument, the computer-assisted surgery system comprising at least one electromagnetic localization system as described above, wherein the first object is an anatomical structure and wherein the end-effector forms the second object. For instance, the processing unit can be adapted to send an instruction to displace the second locating transducer to the motor-driven joint of the robotic arm.

The present invention finally concerns a method for determining the pose of a first object with respect to a second object, wherein at least one first locating transducer is in a fixed position with respect to the first object, wherein at least one second locating transducer is in a fixed position with respect to the second object, wherein at least one of the locating transducer is a transmitter adapted to emit at least one electromagnetic field and the other of the locating transducers is a receiver adapted to receive and measure the electromagnetic field emitted by the transmitter, the method comprising the steps of :
a. determining the pose of the first locating transducer with respect to the second locating transducer based on the measurement of the magnetic field emitted by the transmitter,
b. calculating an indicator related to the accuracy of the measurement of the magnetic field
c. comparing the calculated indicator with a predefined threshold,
d. determining the pose of the first object with respect to the second object based on the determined pose of the first locating transducer with respect to the second locating transducer when the calculated value is below the predefined threshold and
e. generate an instruction to displace at least one of the locating transducers when the calculated value exceeds the predefined threshold, such instruction being executable manually or automatically.

### BRIEF DESCRIPTION OF THE DRAWINGS

Other features and advantages of the invention will appear in the following description. Embodiments of the invention will be described with reference to the drawings, in which
- Figure 1 illustrates, schematically, an electromagnetic localization system according to an embodiment of the invention;
- Figure 2 illustrates, schematically, the electromagnetic localization system according to a first variant of the embodiment illustrated on figure 1;
- Figure 3 illustrates, schematically, the electromagnetic localization system according to a second variant of the embodiment illustrated on figure 1;
- Figure 4 illustrates, schematically, a method to determine the pose of a first object with respect to a second object according to the invention;
- Figures 5 to 8 illustrate a computer-assisted surgery system according to an embodiment the invention illustrated in different situations, said computer-assisted surgery system comprising at least one electromagnetic localization system of the invention.

### DETAILED DESCRIPTION OF THE INVENTION

In the present document, the word "pose" is defined as the position and/or orientation of an object with respect to another object. It can for instance be represented as a transform matrix between a reference system attached to a first object and a reference system attached to the other object.

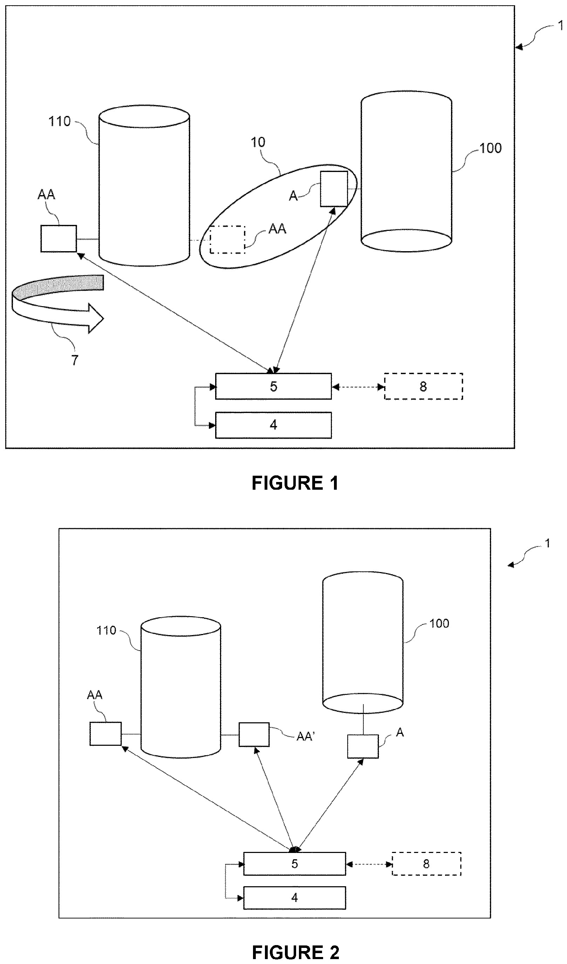

**Figure 1** illustrates, schematically, an electromagnetic localization system 1 according to an embodiment of the invention. The electromagnetic localization system 1 may for instance be used in a medical environment, in various types of medical interventions, including surgery and interventional radiology. Obviously, this is only an example of potential application of the electromagnetic system 1 of the invention, but such system could be used in different applications within the context of the invention.

As illustrated, the electromagnetic localization system 1 can comprise a storage unit 4 configured for storing at least signals and/or data, and at least one processing unit 5 for processing those signals and/or data images in particular in a method to determine the poses of various objects in the surgical room, to facilitate the navigation of a medical instrument (see hereafter). Furthermore, the electromagnetic localization system 1 can be associated with one or several display(s) for the display of images or other parameters that the practitioner may need, for purposes of displaying the relative poses of the tracked objects to the practitioner.

By display we mean any suitable device for displaying information to the practitioner: means suitable for augmented reality, computer screen, means for projecting images, etc. In any case, the invention cannot be limited to a particular display.

The electromagnetic localization system 1 can for instance be used to facilitate the gesture of the practitioner or control the movement of a robot when he/it manipulates an instrument inserted into a patient by locating, with accuracy, the instrument during the navigation, relative to the patient. The electromagnetic localization system 1 may use one or several locating transducer(s) fixed to a patient fiducial for tracking purposes.

To localize a first object 100 relative to a second object 110 in a reference system associated with one of the objects, the electromagnetic localization system 1 further comprises at least one locating unit 10, which comprises at least two locating transducers A, AA: at least one magnetic field transmitter and at least one magnetic field receiver. According to the invention, a first locating transducer A is adapted to be locked, at least temporarily, in a fixed position with respect to the first object 100 while a second locating transducer AA is adapted to be locked, at least temporarily, in a fixed position with respect to the second object 110. As detailed below, the first object can for instance be an anatomical structure of a patient and the second object can be an end-effector of a robotic arm. Obviously, those are only examples, and the first and second objects could be different from what has just been described within the scope of the invention.

The magnetic field receiver is configured to receive and measure the magnetic field emitted by the magnetic field transmitter for the purpose of determining the orientation and/or position of this magnetic field transmitter with respect to the magnetic field receiver.

Subsequently, the term "locating transducer" represents either a magnetic field receiver or a magnetic field transmitter. The magnetic field transmitter(s) and the magnetic field receiver(s) are each associated with an object. In the present document, the words "locating transducer" and "transducer" are used without any distinction.

In the description hereafter, a magnetic field receiver is also called a magnetic receiver or receiver, and a magnetic field transmitter is also called a magnetic transmitter or transmitter.

"Location of an object" or "localization of an object" means that it is possible to determine the pose of the concerned object, i.e. the position of the object for example carrying a magnetic transmitter, as well as its orientation or its inclination with respect to another object, from magnetic data transmitted by said magnetic field transmitter and measured by a magnetic field receiver carried by said other object. In the present case, as will be seen later, said object can be fixed or mobile.

Moreover, in the present description, the use of the word "magnetic" can be replaced by the word "electromagnetic" in the sense that the source - the magnetic transmitter - can be electromagnetic technology, and the magnetic receiver can also be electromagnetic technology.

A transmitter may comprise at least one transmitting coil, and a receiver may comprise at least one receiver coil. Generally, the transmitter comprises a number Ne (Ne≥1) of transmitter coils rigidly connected to each other and oriented along distinct axes. In the following specification, the words "transmitting coil", "emission coil" and "transmitter coil" are used without any distinction, as well as the words "receiver coil", "reception coil" and "receiving coil".

We consider here, by way of example, the case of a system in which the transmitter consists of three emission coils oriented along three distinct axes, for example along the three axes of an orthogonal reference frame. These three axes of the respective magnetic moments of the coils can be confused with the axis of the winding of the coils in the case of a winding orthogonal to the winding axis. They may, however, be different if the winding is not 90 °, for example 45 °.

When a voltage is imposed on the terminals of a transmitting coil, an electric current flows in the transmitting coil which then generates a magnetic field proportional to the current flowing through it and the shape of which depends on the characteristics of the coil (orientation, magnetic moment, shape ....).

A voltage source may, for example, be used to impose a voltage across the transmitting coil resulting in the creation of a current. This voltage may, for example be sinusoidal. The Ne transmitting coils may be subjected to different frequency voltages. Each transmitting coil can also be subjected, simultaneously or not, to at least two different frequencies voltages. In other words, the transmitter comprises Ne transmitting coils adapted to work at Nf frequencies, with Nf ≥ Ne.

Likewise, the receiver generally comprises a number Nr of receiving coils (rigidly connected to each other and oriented along distinct axes). Alternatively, the receiver can comprise a number Nr of magneto-resistive sensors, such as anisotropique magnetoresistance sensors, global magneto-resistive sensor, tunnel magneto-resistive sensors or SQUID (superconducting quantum interference device) sensors. As known in the art, such magneto-resistive sensors comprise a resistive element with a resistance proportional to a value of the magnetic field they receive. Obviously, the receiver can comprise any other known electromagnetic receiver within the scope of the invention.

Here, by way of example, the case of a system in which the receiver is consisting of three receiving coils oriented along three distinct axes, for example along the three axes of an orthogonal reference.

In the presence of a variable magnetic field, a voltage proportional to the variation in the flux of the magnetic field appears in the reception coil. By measuring the voltage at the terminals of the reception coil, using a voltmeter or other similar means, or by measuring the current passing through the reception coil by an ammeter or other similar means, it is possible to determine the magnetic field to which the reception coil is subjected, provided that the characteristics of the reception coil are known, which include notably the magnetic moments of the reception coils, or at least the ratio of the magnetic moments of the reception coils.

The transmitter and the receiver are connected to a processing unit, including for example a microprocessor connected on one hand to the generator of the transmission device and on the other hand to the reading device of the reception device. The processing unit is configured to process the signals transmitted and received, and thus makes it possible to determine from the latter the pose of the receiver relative to the transmitter.

The processing unit receives information concerning the characteristics of the excitation signals applied to the transmission coils as well as information concerning the characteristics of the signals passing through the reception coils (representative of the field received by the reception coil). From the intensity of the fields captured by the reception coils, the processing device is adapted to determine the pose of the set of reception coils with respect to the set of transmission coils.

More particularly, the processing unit determines (Ne x Nr) field values respectively corresponding to the field received by the receiver coil from the transmitter coil.

From these field values, the processing unit is adapted to calculate the spatial position and orientation coordinates of the set of receiver coils with respect to the transmitter coils.

As previously explained, during a phase of use of the electromagnetic localization system, the processing device uses known characteristics of the transducers and the characteristics of the field received by the receiver to determine the relative poses of the transducers.

According to the invention, the processing unit 5 is also adapted to calculate an indicator related to the accuracy of the measurement of the magnetic field emitted and received, respectively, by the first and second locating transducers A, AA of the locating unit 10. The processing unit 5 can then compare this calculated indicator with a predefined threshold to determine if the measurement is reliable or if it is disturbed, for instance by disturbing material(s) which can be in the vicinity of said locating unit. Especially, the threshold is set such that the measurement of the magnetic field is considered reliable if the calculated indicator is below such threshold.

Advantageously, the processing unit 5 can optionally be adapted to determine a working distance of the concerned locating unit, i.e. a distance measured between the first and the second locating transducers A, AA. The processing unit 5 can then compare such determined distance with a predefined threshold to determine if the measurement is reliable. For instance, this threshold can be set at 60 mm, advantageously 50 mm.

Therefore, when the calculated indicator related to the accuracy of the measurement and/or the determined working distance is/are below their respective thresholds, the processing unit 5 is adapted to determine the pose of the first object 100 with respect to the second object 110 based on the determined pose of the first and second locating transducers A, AA with respect to one another. When said calculated indicator and/or said working distance are/is above said thresholds, the processing unit 5 is adapted to generate an instruction to displace at least one of the locating transducers. As such, it is understood that said locating transducer is only temporarily fixed in position with respect to the concerned object. For instance, figure 1 illustrates a situation wherein the second locating transducer AA is adapted to be displaced, as illustrated by the arrow 7 and by the locating transducer AA represented with dotted lines. According to the invention, this instruction can be executed manually or automatically.

For instance, the electromagnetic localization system 1 can comprise an alert device 8 adapted to receive said generated instruction, the alert device 8 being then adapted to provide information to the user of the system that he/she needs to displace at least one of the locating transducers to improve the accuracy of the measurement. For instance, the alert device 8 can be a display adapted to display a message, an image, a colored indicator, or it can be adapted to vibrates or to emit sound in order to provide said information to the user. This alert device 8 is represented with dotted lines on figure 1 as it is optional. Obviously, such an alert device 8 can implement any other means to provide said information to the user of the system.

Optionally, the instruction sent to the alert device can also encompass an information indicating the direction along which the transducer must be displaced to improve the accuracy of the measurement. For instance, a model can be registered in the processing unit which can then be adapted to measure the deviation of the magnetic field and to determine based on said model and said deviation, the direction along which the transducer must be displaced to improve the accuracy of the measurement. Alternately, the processing unit can be adapted to calculate the indicator in real time when the locating transducer is displaced and to determine if the indicator increases or decreases and to, subsequently indicate a direction along which the locating transducer must be displaced in order to improve the measurement, i.e. a direction permitting to decrease the indicator.

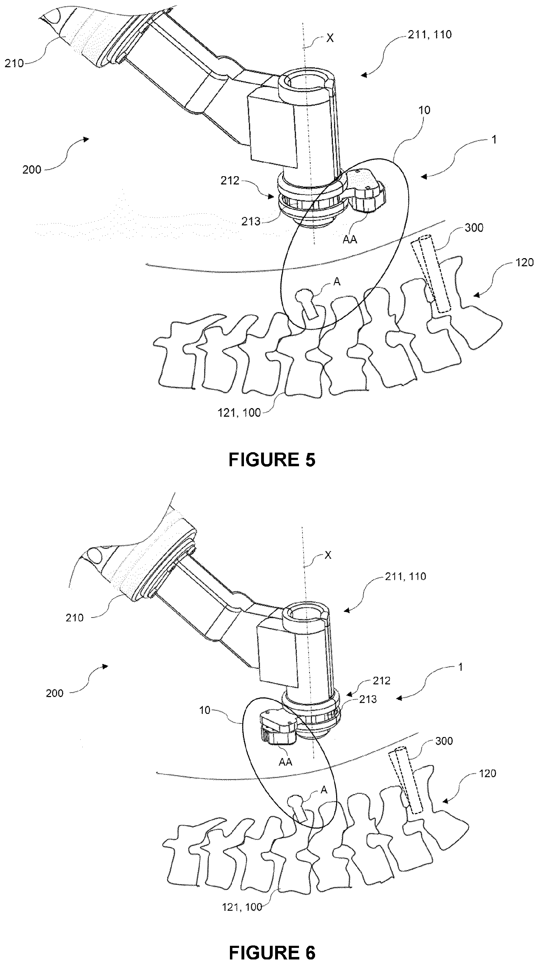

As mentioned above, figure 1 illustrates an embodiment wherein the second locating transducer AA is movably attached to the second object 110. For instance, this second locating transducer AA can be mounted on a guide - for instance illustrated on figures 5 to 8 - fixedly attached to the second object 110. According to the invention, such guide comprises at least one locking device, or one indexing device adapted to lock the pose of the second locating transducer AA with respect to the second object. Using a locking device allows a wider range of different potential poses for the concerned locating transducer while using an indexing device permits to know more easily the exact pose of said locating transducer with respect to the associated object. The locking device can for instance be realized as a locking lever, or as a clamping means. This aspect of the invention will be detailed below.

Optionally, the second locating transducers AA can be motor-driven and the processing unit 5 can then be adapted to send the generated instruction to such motor so that the motor-driven locating transducer is automatically displaced.

According to another embodiment described below, the second object 110 can be formed as an end-effector of a robotic arm, said end-effector being adapted to hold a surgical guide or a surgical instrument. Such a robotic arm encompasses at least two arm segments linked to one another thanks to at least one motor-driven joint. According to this other embodiment, the processing unit can be adapted to send the instruction to the motor-driven joint, such joint being then moved in order to displace the second locating transducer. Advantageously, provided that the robotic arm presents enough degrees of freedom, such a configuration permits to displace the second locating transducer to improve the localization accuracy but without displacing the end-effector, nor the surgical instrument or the surgical guide held by said end-effector.

Once that the locating transducer has been displaced, either manually or automatically, the processing unit 5 can be adapted to determine the new pose of the locating transducers with respect to one another, then to calculate the corresponding indicator of accuracy and finally to determine the pose of the first object with respect to the second object based on the determined pose of the locating transducers if the calculated indicator is below the threshold, and generate another instruction to displace one of the locating transducers when the indicator is above said threshold. Again, this other instruction can be executed manually or automatically. Obviously, those steps can be repeated as long as the calculated indicator exceeds the predefined threshold. Obviously, all these steps can also be carried out for the evaluation of the working distance of the locating unit, within the scope of the invention.

**Figure 2** illustrates a first variant of the embodiment illustrated on figure 1. This variant differs from the embodiment that has just been described, in that the system 1 comprises at least two second locating transducers AA, AA' i.e. at least two locating transducers are adapted to be locked in a fixed position with respect to the second object 110, at least temporarily. According to this variant, the processing unit 5 is adapted to determine the pose of the first locating transducer A with respect to both the second locating transducers AA, AA' and to determine subsequently, the indicators related to the accuracy of each measurement. If both indicators exceed the predefined threshold, the processing unit is adapted to generate an instruction to displace at least one of the locating transducers. As previously described, such an instruction can be executed manually or automatically. If at least one of the indicators is below the predefined threshold, the processing unit is adapted to determine the pose of the first and second object with respect to each other based on the measurement associated with the lowest indicator.

Again, according to this first variant, the processing unit can be adapted to determine the working distance for each pair of first and second locating transducers of the locating unit and to generate an instruction to displace at least one of the locating transducers when each working distance exceeds the predefined threshold. As previously mentioned, such predefined threshold can be set at 60 mm, advantageously 50 mm.

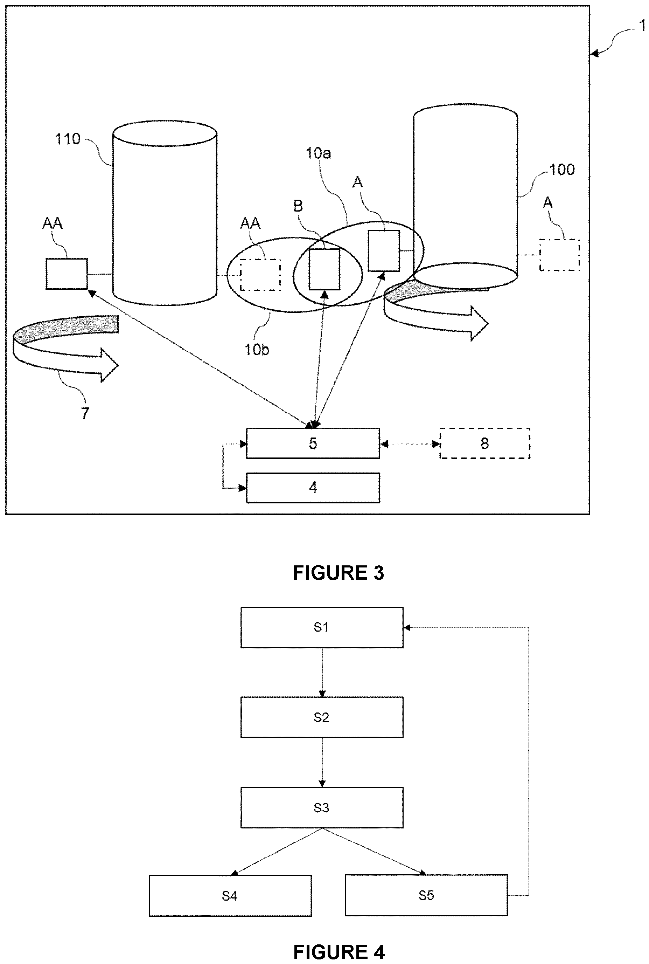

**Figure 3** illustrates a second variant of the embodiment illustrated on figure 1. According to this second variant, the electromagnetic localization system 1 comprises two locating units 10a, 10b, each locating unit comprising at least two locating transducers wherein at least one transmitter adapted to emit at least one magnetic field, and one receiver adapted to receive and measure the magnetic field emitted by the transmitter. Especially, figure 3 illustrates a situation wherein at least one of the locating transducers B is shared by both the locating units 10a, 10b. This shared locating transducer B thus forms a relay which helps determine the poses of the two other locating transducers A, AA. Indeed, using such a shared locating transducer B permits to decrease the respective working distances of each locating unit, thus improving the accuracy of the measurement of the corresponding magnetic fields. In the present document, the "working distance" of a locating unit designates a distance measured between the transmitter and the receiver of one same locating unit. The smaller this working distance is, the lower the risk of having a disturbed magnetic field is. As previously mentioned, this working distance can be monitored by the processing unit 5 and be considered to generate the instruction to displace at least one of the locating transducers. This working distance can also be taken into account in the calculation of the indicator related to the accuracy of the measurement of the magnetic field(s).

Especially, according to this second variant of the embodiment of figure 1, the processing unit 5 is thus adapted to:
a. determine the pose of the locating transducers A, B ; B, AA forming each locating unit 10a, 10b with respect to one another,
b. calculate the indicator related to the accuracy of the measurement of the magnetic field for both locating units 10a, 10b,
c. compare the calculated indicators with the predefined threshold,
d. determine the pose of the first object 100 with respect to the second object 110 based on the determined poses of the locating transducers of each locating unit when both indicators are below the predefined threshold, and
e. generate an instruction to displace at least one of the locating transducers when at least one of the indicators exceeds the predefined threshold.

For instance, the processing unit can be adapted to generate the instruction to displace the shared locating transducer B when both indicators exceed the predefined threshold, such instruction being executable manually or automatically. The processing unit can also be adapted to generate the instruction to displace the shared locating transducer or one of the not-shared locating transducers when only one of said indicators exceeds the predefined threshold. Obviously, if the instruction concerns the not-shared locating transducer, it is directed to the not-shared locating transducer of the locating unit associated with the indicator exceeding the predefined threshold. As previously explained, such instruction can be executed manually or automatically.

Obviously, those are only examples of how to carry the invention, but the electromagnetic localization system 1 could comprise more than two locating units, with more or less than one shared locating transducer within the scope of the invention.

**Figure 4** generally illustrates a method of the invention for determining the pose of the first object with respect to the second object, wherein at least one first locating transducer is in a fixed position with respect to the first object, wherein at least one second locating transducer is in a fixed position, at least temporarily, with respect to the second object, wherein at least one of the locating transducer is a transmitter adapted to emit at least one electromagnetic field and the other of the locating transducers is a receiver adapted to receive and measure the electromagnetic field emitted by the transmitter, the method comprising the following steps:
S1: determining the pose of the first locating transducer with respect to the second locating transducer based on the measurement of the magnetic field emitted by the transmitter,
S2: calculating an indicator related to the accuracy of the measurement of the magnetic field
S3: comparing the calculated indicator with a predefined threshold,
S4: determining the pose of the first object with respect to the second object based on the determined pose of the first locating transducer with respect to the second locating transducer when the calculated value is below the predefined threshold and
S5: generating the instruction to displace at least one of the locating transducers when the calculated value exceeds the predefined threshold, such instruction being executable manually or automatically.

As illustrated on figure 4, the method can be repeated after the displacement of the locating transducer to ensure that such displacement improves the accuracy of the measurement of the magnetic field.

Optionally, the method can further comprise an additional step wherein the processing unit is adapted to determine the working distance of each locating unit, to compare these working distances with a predefined threshold and to generate an instruction to displace at least one of the locating transducers when said working distance exceeds said predefined threshold. As previously mentioned, such threshold can be set at 60 mm, advantageously, at 50 mm.

The calculated indicator can be of different kinds within the scope of the invention. We are now going to describe some of the indicators which can be used but it is understood that these are only examples and that any other known indicator can be used without departing from the scope of the invention.

For instance, the processing unit can be adapted to implement the least squares optimization method to determine the pose of the locating transducers with respect to one another, and the indicator related to the accuracy of the measurement can then be calculated based on the residual errors of such square optimization method and on the working distance of the concerned locating unit.

According to an aspect of the invention, the calculated indicator can be obtained based on the following reasoning.

Let X, a state vector representing the state of the system for a specific pose and Y a measurement in such state. We can thus create a model function h, "Eₓ → E_{y}", which gives a prediction of the measurement in the concerned state. Then, an error between the prediction (H) and the real measurement (Y) can be determined. Let e represent such error, then if Y'=h(X), e=Y-Y'. When several measurements are realized, the residual error can thus be modelized by the following:
R(X) = |Y-h(X)|², wherein R is a scalar which is related to the quality of the model function h(X) with respect to the measurement of Y in X.

Consequently, it is understood that the lower the residual error is, the most accurate the measurement is. By setting a threshold, we can thus ensure that the least accurate measurements are not considered in the determination of the pose of the first object with respect to the second object. This indicator can also be calculated for two rigidly linked transducers.

According to an aspect of the invention, the system can comprise an additional transducer rigidly linked to the receiver of the at least one locating unit, the processing unit being adapted to monitor the pose of said receiver with respect to the additional transducer. As the receiver and the additional transducer are rigidly linked to each other, their relative pose should remain constant. Consequently, if a variation of such relative pose is detected and exceeds a predefined threshold, it means that the magnetic field is disturbed and the processing unit is then adapted to generate the instruction to displace one of the locating transducers of the concerned locating unit.

According to another alternative, the processing unit can be adapted to monitor the phase of the received signal, i.e. the phase of the magnetic field received and measured by the receiver of a locating unit. This phase should remain identical at any time, so if this phase varies, it means that the electromagnetic field is disturbed. Consequently, a variation value can be defined such that is the phase varies more than said variation value, the processing unit is adapted to determine that the magnetic field is disturbed and to instruct the displacement of at least one of the transducers.

According to another variant, the objects linked to the transducers can also be linked to inertial sensors, such as accelerometer or gyroscope, adapted to detect and measure the displacement of said object. The information obtained by the measurement of the magnetic fields and the information obtained by the measurements realized thanks to the inertial sensors can thus be compared to determine whether the magnetic field is disturbed or not. As previously described, if the difference between these information exceeds a predefined threshold, it means that the magnetic field is disturbed and the processing unit is then adapted to generate the instruction to displace one of the locating transducers. Alternatively, the inertial sensors can be replaced by optical sensors or by an ultra-white band time of flight systems.

According to another aspect of the invention, the electromagnetic localization system can comprise at least one additional transducer fixed in position in the system referential, such additional transducer being a receiver adapted to receive and measure the magnetic field emitted by the at least one transmitter of the system. The processing unit is thus adapted to determine the pose of such additional transducer and to compare said obtained pose with its registered pose. If the difference between those poses exceeds a predefined threshold, it means that the magnetic field is disturbed, and the processing unit is then adapted to instruct the displacement of at least one of the other transducers.

According to another embodiment, the electromagnetic localization system can comprise at least two additional transducers arranged in a solid device of a known geometry. The processing unit is thus adapted to monitor the pose of such additional transducers and to determine whether they are indeed in the defined geometry or not. Again, if the difference between the determined pose and the registered one exceeds a predefined threshold, it means that the magnetic field is disturbed and the processing unit is then adapted to generate the instruction to displace one of the locating transducer associated with the objects to be localized.

The present invention also concerns a computer assisted surgery system 200, for instance partially illustrated on **figures 5 and 6****.** Especially, figures 5 and 6 illustrate a situation wherein the computer assisted surgery system 200 is used to assist a surgical intervention on a rachis 120. Obviously, this is only an example and the system could be used in a wide variety of interventions. As illustrated, such computer-assisted surgery system 200 comprises at least one robotic arm 210 which extends between a base - not shown here - and an end-effector 211 adapted to hold a surgical tool or a surgical guide. Preferably, the surgical tool is a linear tool such as a trocar, a needle, a drill, a burr, a screwdriver or equivalent. Figures 5 and 6 especially illustrate a main axis of extension X of such a linear tool. As previously mentioned, the robotic arm comprises several segments, each segment being separated from the adjacent segment by a joint.

The illustrated computer-assisted surgery system further comprises the electromagnetic localization system 1 described above. According to the illustrated embodiment, the first object 100 is an anatomical structure, especially a vertebra 121 of a patient, and the second object 110 is formed as the end-effector 211 of the robotic arm 210.

In order to perform the intervention, the poses of the surgical tool or surgical guide with respect to the anatomical structure of interest - here the vertebrae - must be precisely known at any time. This is achieved thanks to the electromagnetic localization system 1 of the computer-assisted surgery system of the invention.

If the user of the system needs to intervene on one of the vertebra of the patient, the first locating transducer A is attached to the concerned vertebra 121 and the second locating transducer AA is attached to the end-effector 211 of the robotic arm 210. Obviously, if the user needs to intervene on several vertebrae of the patient, several locating transducers can be attached to the concerned vertebras. **Figures 5 and 6** especially illustrate an embodiment of the invention wherein the second locating transducer AA is mounted on a guide 212 fixedly attached to the end-effector 211. For instance, this guide 212 can be glued, screwed or taped to the end-effector 211. Obviously, these are only example on how to fixedly attach the guide on the end-effector 211 and any other means could be used within the scope of the invention. Particularly, according to the illustrated embodiment, the guide 212 comprises an indexing device 213, thus allowing the positioning of the second locating transducer AA only on a limited number of poses. Those poses can for instance be recorded in the storage unit of the electromagnetic localization system 1 so that the user of the system can easily indicate to the processing unit, the exact position of the second locating transducer AA with respect to the end-effector 211. The pose of the end-effector 211 with respect to the base of the computer-assisted surgery system can be determined by the processing unit 5, for instance thanks to encoders associated with the joints of the robotic arm 210.

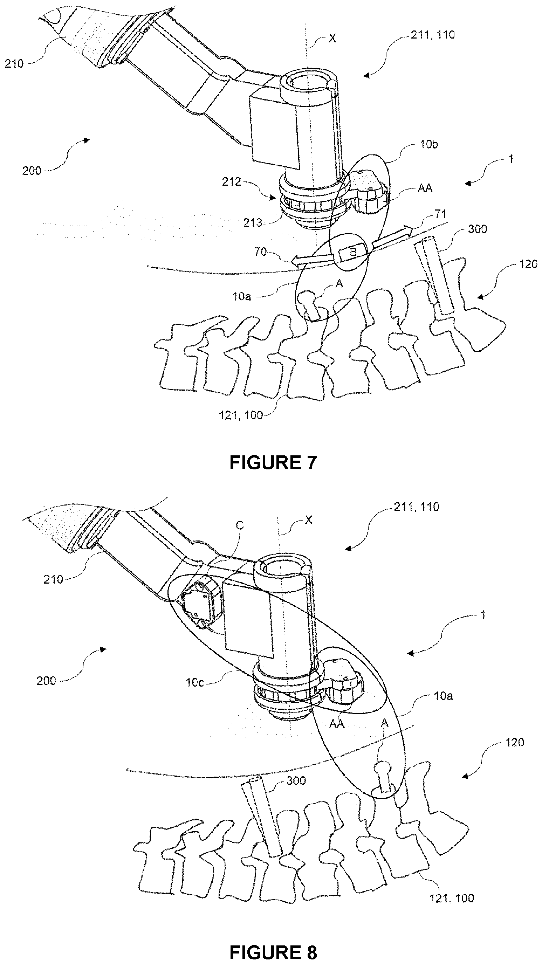

Alternatively, as for instance illustrated on **figure 8****,** an additional transducer C can be rigidly attached to the robotic arm 210, near the end-effector 211, thus forming another locating unit 10c together with the locating transducer AA locked in position with respect to the end-effector 211. As illustrated, the additional transducer C is fixed in position with respect to the end-effector 211. Thus, once the relative pose of the additional locating transducer C with respect to the locating transducer AA locked in position with respect to the end-effector is known, the pose of the locating transducer AA locked in position with respect to the end-effector 211 can be determined. In this alternative, it is understood that the indexing means 213 could be replaced by a locking device, such as a locking lever or clamping device for instance.

**Figure 5** illustrates a first situation where a first measurement of the magnetic field is realized and the processing unit 5 thus calculates the indicator related to the accuracy of such measurement. If this indicator exceeds the predefined threshold, at least one of the transducers is displaced, manually or automatically, as previously described. **Figure 6** thus illustrates a situation where the second locating transducer AA has been displaced. Once the displacement is achieved, the magnetic field can be measured a second time, and the processing unit can calculate the indicator associated with this second measurement. If the indicator still exceeds the predefined threshold, one of the locating transducers can be displaced again but if the indicator is below said predefined threshold, the processing unit is adapted to determine the pose of the locating transducers A, AA with respect to one another and then to determine the pose of the first and second object 100, 110 with respect to each other. As the pose of the end-effector 211, here forming the second object, can be determined with respect to the base of the computer-assisted surgery system, the pose of the concerned vertebra, here forming the first object, can also be determined with respect to the base.

**Figure 7** finally illustrates, schematically, an application of the variant illustrated and described above with reference to figure 3. As previously explained, the system 1 here comprises two locating units 10a, 10b with a shared transducer B. Thus, an indicator of the accuracy is calculated for the measurement realized for each locating unit. If both indicators indicate that the magnetic field is disturbed, the shared locating transducer B can be displaced in order to improve both measurements, as schematically illustrated with arrows 70, 71. For instance, this shared locating transducer B can be removably taped to the skin of the patient, or it can be mounted on a movable device itself attached to the skin of the patient as long as its pose can be fixed with respect to the anatomical structure to localize. If only one of the indicators is above the predefined threshold, then one or both of the shared transducer B and the locating transducer AA attached to the end-effector can be displaced in order to improve the measurement.

Advantageously, as schematically illustrated with dotted lines on figures 5 to 8, the electromagnetic localization system of the invention permits to consider the presence of implants 300 which have already been implanted in one of the patient's vertebrae, for instance during a previous surgery, or during the ongoing surgery. Such implants 300 can for instance be made of metal and thus disturb the measurement of the magnetic fields. As explained in the present document, the invention advantageously permits to determine if those implants actually disturb the measurement of the magnetic field or if they can be ignored in the calculation of the relative pose of the concerned objects.

Obviously, the invention cannot be limited to the means and configuration described and illustrated herein, and it also extends to any equivalent means or configurations and to any technically operative combination of such means. In particular, the shape and arrangement of the locating unit and of the guide can be modified insofar as they fulfil the functionalities described in the present document.

## Claims

1. An electromagnetic localization system (1) comprising at least one locating unit (10a, 10b) and one processing unit (5), the locating unit (10a, 10b) comprising at least two locating transducers (A, AA, AA' B) wherein at least one locating transducer (A, AA, AA', B) is a transmitter adapted to emit at least one magnetic field and at least one locating transducer (A, AA, AA' B) is a receiver adapted to receive and measure the magnetic field emitted by the transmitter, wherein at least one first locating transducer (A, B) is adapted to be locked in a fixed position with respect to a first object (100) and at least one second locating transducer (AA, AA' B) is adapted to be locked in a fixed position with respect to a second object (110), the processing unit (5) being configured to:
a. determine a pose of the first locating transducer (A, B) with respect to the second locating transducer (AA, AA', B) based on the measurement of the magnetic field emitted by the transmitter of the locating unit,
b. calculate an indicator related to the accuracy of the measurement of the magnetic field,
c. compare the calculated indicator with a predefined threshold,
d. determine the pose of the first object (100) with respect to the second object (110), based on the determined pose of the transducers when the calculated indicator is below the predefined threshold, and
e. generate an instruction to displace at least one of the locating transducers when the calculated indicator exceeds the predefined threshold, such instruction being executable manually by a user of the system or automatically by an actuator.

2. The electromagnetic localization system (1) according to the preceding claim, comprising at least one alert device (8) and wherein the processing unit (5) is adapted to send the instruction to displace one of the locating transducers (A, AA, AA', B) to the alert device (8), the execution of such instruction resulting in that the alert device (8) provides an information to the user according to which one of the locating transducers must be displaced.

3. The electromagnetic localization system (1) according to any of the preceding claims, wherein the second locating transducer (AA, AA') is movably attached to the second object (110).

4. The electromagnetic localization system (1) according to the preceding claim, wherein the second locating transducer (AA, AA') is mounted on a guide (212) fixedly attached to the second object (110), and wherein the system (1) comprises at least one locking device adapted to lock the pose of the second locating transducer with respect to the second object (110).

5. The electromagnetic localization system (1) according to any of claims 1 to 4, wherein the second locating transducer (AA, AA') is mounted on a guide (212) fixedly attached to the second object (110), the guide (212) comprising an indexing device (213) adapted to lock the pose of the second locating transducer in one of a number of determined poses with respect to the second object (110).

6. The electromagnetic localization system (1) according to any of claims 4 to 6, wherein the second locating transducer (AA, AA') is motor-driven, the processing unit (5) being adapted to send the instruction to displace one of the locating transducers to the motor driving the second locating transducer, the execution of the instruction resulting in a displacement of the second locating transducer (AA, AA').

7. The electromagnetic localization system (1) according to any of the preceding claims, comprising at least two locating units (10a, 10b), each locating unit comprising at least two locating transducers (A, AA, AA', B), wherein at least one locating transducer is a transmitter adapted to emit at least one magnetic field and at least one locating transducer is a receiver adapted to receive and measure the magnetic field emitted by the transmitter, wherein the two locating units comprise at least one shared locating transducer (B) adapted to be locked in a fixed position with respect to both objects (100, 110) and wherein the processing unit (5) is adapted to
a. determine a pose of the locating transducers of each locating unit with respect to one another based on the measurement of the magnetic field emitted by the transmitter of the concerned locating unit,
b. calculate one indicator related to the accuracy of the measurements of the magnetic field for each measurement,
c. compare each of the calculated indicators with a predefined threshold,
d. determine the pose of the first object (100) with respect to the second object (110), based on the determined pose of the transducers when the calculated indicators are both below the predefined threshold, and
e. generate an instruction to displace at least one of the locating transducers when at least one of the calculated indicators exceed the predefined threshold, such instruction being executable manually by a user of the system or automatically by an actuator.

8. The electromagnetic localization system (1) according to the preceding claim, wherein the processing unit (5) is adapted to generate an instruction to displace the shared locating transducer (B) when both calculated indicators exceed the predefined threshold.

9. The electromagnetic localization system (1) according to any of the preceding claims, wherein at least one of the locating units comprises at least two second locating transducers adapted to be locked in position with respect to the second object (110), the two second locating transducers being either transmitters adapted to emit the electromagnetic field then received and measured by the first locating transducer or receivers adapted to receive and measure the electromagnetic field emitted by the first locating transducer, the processing unit (5) being adapted to:
a. determine the pose of the first locating transducer with respect to each of the second locating transducers,
b. calculate at least one indicator related to the accuracy of the measurements of the magnetic fields for each determined pose,
c. compare each calculated indicator to the predefined threshold
d. determine the pose of the first object (100) with respect to the second object (110) when at least one of the calculated indicators is below the predefined threshold, based on the determined pose of the first locating transducer with respect to the one of the second locating transducers for which the calculated indicator related to the accuracy of the measurement of the magnetic field is the lowest
e. generate an instruction to displace at least one of the locating transducers when all the calculated indicators exceed the predefined threshold, such instruction being executable manually or automatically.

10. The electromagnetic localization system (1) according to any of the preceding claims, wherein the processing unit (5) is further configured to:
a. determine a distance between the first locating transducer (A, B) and the second locating transducer (AA, AA', C) of at least one locating unit (10a, 10b),
b. compare the determined distance measured between the first locating transducer and the second locating transducer of the locating unit with a predetermined threshold,
c. generate an instruction to displace at least one of the locating transducers when the determined distance exceeds the predetermined threshold, such instruction being executable manually by a user of the system or automatically by an actuator.

11. Computer-assisted surgery system (200), comprising at least one robotic arm (210) with at least one motor-driven joint formed between two adjacent segments, the robotic arm (210) extending between a base and an end-effector (211), such end-effector being adapted to hold a surgical guide (212) and/or a surgical instrument, the computer-assisted surgery system (200) comprising at least one electromagnetic localization system (1) according to any of the preceding claims, wherein the first object (100) is an anatomical structure and wherein the end-effector (211) forms the second object (110).

12. Computer-assisted surgery system (200) according to the preceding claim, wherein the processing unit (5) is adapted to send an instruction to displace the second locating transducer (AA, AA', B) to the motor-driven joint of the robotic arm (210).

13. Method for determining the pose of a first object (100) with respect to a second object (110), wherein at least one first locating transducer is in a fixed position with respect to the first object (100), wherein at least one second locating transducer is in a fixed position with respect to the second object (110), wherein at least one of the locating transducer is a transmitter adapted to emit at least one electromagnetic field and the other of the locating transducers is a receiver adapted to receive and measure the electromagnetic field emitted by the transmitter, the method comprising the steps of:
a. determining the pose of the first locating transducer with respect to the second locating transducer based on the measurement of the magnetic field emitted by the transmitter,
b. calculating an indicator related to the accuracy of the measurement of the magnetic field
c. comparing the calculated indicator with a predefined threshold,
d. determining the pose of the first object (100) with respect to the second object (110) based on the determined pose of the first locating transducer with respect to the second locating transducer when the calculated value is below the predefined threshold and
e. generate an instruction to displace at least one of the locating transducers when the calculated value exceeds the predefined threshold, such instruction being executable manually or automatically.
